# FASCICULE DE BREVET EUROPEEN

(11) **EP 0 458 696 B1**
(45) Date de publication et mention de la délivrance du brevet: **03.01.1996**
(21) Numéro de dépôt: 91401311.5
(22) Date de dépôt: 21.05.1991
(51) Int. Cl.: A61K 31/44

(54) **Utilisation d'une 1-(2-naphtyléthyl)-4-(3-trifluorométhylphényl)-1,2,3,6-tétrahydropyridine pour la préparation de médicaments destinés au traitement de troubles cérébraux et neuronaux**
Verwendung von 1-(2-Naphthylethyl)-4-(3-trifluoromethylphenyl)-1,2,3,6-tetrahydropyridin zur Herstellung eines Medikaments zur Behandlung von zerebralen und neuronalen Krankheiten
Use of 1-(2-naphthylethyl)-4-(3-trifluoromethylphenyl)-1,2,3,6-tetrahydropyridine for the preparation of medicaments for the treatment of cerebral and neuronal diseases

(30) Priorité: 22.05.1990 FR 9006399
(43) Date de publication de la demande: 27.11.1991
(60) Demande divisionnaire: 94403014.7
(73) Titulaire: SANOFI, F-75008 Paris (FR)
(72) Inventeur: Coude, François Xavier, F-31500 Toulouse (FR); Fournier, Jacqueline, F-31830 Plaisance du Touch (FR); Guzzi, Umberto, I-20148 Milano (IT)
(74) Mandataire: Gillard, Marie-Louise

(56) Documents cités:
- EP-A- 0 101 381
- JOURNAL OF NEUROCHEMISTRY, vol. 41, no. 1, juillet 1983, Internatl. Society for Neurochemistry, Ravens Press, New York, NY (US); D.M. BOWEN et al., pp. 266-272
- ARZNEIMITTEL-FORSCHUNG, vol.39, no. 8a, 1989; C.G. GOTTFRIES, pp. 1025-1030
- ANNALS OF THE NEW YORK ACADEMY OF SCIENCES, vol. 600, 1990, 'The Neuropharmacology of Serotonin', New York Academy of Sciences, New York, NY (US); A.J. CROSS, pp. 405-417
- ANN. NEUROL. USA, vol. 22, no. 2, 1987; R.J. D'AMATO et al., pp. 229-236

## Description

La présente invention concerne l'utilisation d'une 1-(2-naphtyléthyl)-4-(3-trifluorométhylphényl)-1,2,3,6-tétrahydropyridine de formule suivante: 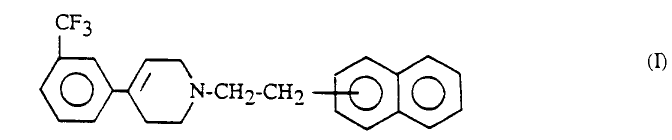
ou de ses sels d'addition avec des acides pharmaceutiquement acceptables, pour la préparation de médicaments destinés au traitement et/ou à la prophylaxie des maladies cérébrales et neuronales.

Plus particulièrement, la présente invention concerne l'utilisation d'un composé de formule (I) ou de ses sels d'addition pharmaceutiquement acceptables pour la préparation de médicaments destinés au traitement des maladies associées à une dégénérescence neuronale.

Dans la formule (I) ci-dessus, le groupe 1-[4-(3-trifluorométhylphényl)-1,2,3,6-tétrahydropyridinyl]éthyl peut être liée en position 1 ou 2 du naphtalène. Selon un aspect préféré de la présente invention ce groupe est lié en position 2 du naphtalène.

Les composés de formule (I), sous forme de bases libres ou de sels d'addition, ainsi que leur préparation ont été décrits dans la demande de brevet européen EP-A-101381. Parmi les sels d'addition du composé de formule (I) pharmaceutiquement acceptables, on peut citer ceux formés avec les acides minéraux, tels que par exemple les acides chlorhydrique, bromhydrique, phosphorique ou sulfurique, ou avec les acides organiques tels que l'acide acétique, formique. propionique, benzoïque, maléique, succinique, tartrique, fumarique, citrique, glyoxylique, aspartique, méthanesulfonique, éthanesulfonique, benzènesulfonique, p-toluènesulfonique, etc.

Dans la demande de brevet européen citée ci-dessus on signale une activité anorexigène des produits.

On a maintenant trouvé, de façon tout à fait inattendue, que les composés de formule (I) sont en mesure d'exercer une action neurotrophique sur le système nerveux, semblable à celle du facteur de croissance nerveuse (NGF de l'anglais *Nerve Growth Factor*) et peuvent rétablir le fonctionnement des cellules nerveuses endommagées ou présentant des anomalies dans leurs fonctions physiologiques. Cette activité neurotrophique a été d'abord mise en évidence dans un test de neuritogénèse in vitro.

### Evaluation pharmacologique in vitro

Le test de neuritogénèse in vitro a été conduit en utilisant des neurones isolés à partir de dissections de la région septale des embryons de rats selon des procédures conventionnelles qui permettent d'obtenir des suspensions neuronales enrichies (de 95 à 98%). (S.E. Bottenstein: "Growth and differenciation of neural cells in defined media" dans Cell Culture in the Neuroscience, p. 3-43, 1985, Eds: S.E. Bottenstein, G. Sato).

Plus particulièrement, on a prélevé la région septale des embryons de rats âgés de 17 jours sous microscope à dissection en maintenant, pendant toute la durée du prélèvement, les structures à 4°C dans le milieu suivant:

| DME/F₁₂ (v:v) | |
|---|---|
| glucose | 5 % |
| amphotéricine B | 1 % |
| gentamycine | 0,5 % |

Les cellules sont dissociées par traitement pendant 20 minutes, à 37°C, avec tripsine-EDTA, puis centrifugées deux fois et lavées au PBS. La dissociation est complétée à la pipette pasteur dans du milieu de Hanks. Cette étape est suivie par 3 centrifugations. Le culot obtenu a été repris dans un milieu sérique:

| DME/F₁₂ (v:v) | |
|---|---|
| KCl 34 mM | |
| sérum de veau foetal | 5 % |
| sérum de cheval | 5 % |
| glutamine | 0,1 % |
| amphotericine B | 1 % |
| gentamycine | 0,5 % |

Cette suspension cellulaire est placée dans un flacon de culture et maintenue à l'étuve pendant 90 minutes à 37°C sous 5% de CO₂.

Les cellules non nerveuses vont rapidement adhérer sur le plastique du flacon, et la suspension est donc enrichie en neurones (95 à 98%). Ces suspensions ont été centrifugées et les culots repris dans un milieu de culture non sérique (H.W. Muller et N. Seifert, J. Neurosc. Res., 8, 195-204, 1982):

| DME/F₁₂ (v:v) | |
|---|---|
| transferrine | 1 µg/ml |
| tri-iodothyronine | 3 mM |
| insuline | 5 µg/ml |
| hydrocortisone | 20 µM |
| glutamine | 0,1 % |
| amphotericine B | 1 % |
| gentamycine | 0,5 % |

Les neurones ont été mis en culture dans des plaques de 96 puits (5x10⁴ cellules viables par puits).

Chaque puits a été traité à la poly-L-lysine (10 µg/ml) afin de former une matrice indispensable à l'adhésion, la survie et la différenciation des neurones. Des échantillons de 130 µl de milieu non sérique contenant soit les produits à tester de formule (I) aux doses choisies, soit le solvant du produit (diméthylsulfoxyde) aux concentrations correspondantes, ont été distribués dans les puits. Après ensemencement des neurones, les plaques ont été maintenues à l'étuve (37°C - 5% CO₂) pendant 18 heures. Les cellules, après fixation dans un mélange glutaraldéhyde/paraformaldéhyde, ont été comptées de la façon suivante:
- dans chaque puits ensemencé on compte, pour un champ microscopique donné, le nombre total de cellules observées et le nombre de cellules ayant au moins un neurite (neurite = prolongement) ≧ de deux fois le diamètre de la cellule,
- on compte cinq champs par puits et pour chaque dose de produit testé on ensemence deux puits, on obtient donc dix valeurs pour chaque dose,
- les résultats sont exprimés en pourcentage de cellules à neurites sur le nombre total de cellules comptées. Chaque lot est comparé à son contrôle par une analyse non paramétrique de Kruskall-Wallis.

Les résultats obtenus avec le chlorhydrate de la 1-[2-(2-naphtyl)éthyl]-4-(3-trifluorométhylphényl)-1,2,3,6-tétrahydropyridine (Composé A) sont les suivants:

**TABLEAU I**

| | % DE CELLULES A NEURITES |
|---|---|
| Composé A: 2,4 nM | 44,8 ± 3,45 |
| Contrôle (DMSO 10⁻⁶) | 30,2 ± 1,68 |
| Composé A: 24 nM | 39,2 ± 2,39 |
| Contrôle (DMSO 10⁻⁵) | 29,4 ± 1,64 |
| Composé A: 240 nM | 44,1 ± 2,02 |
| Contrôle (DMSO 10⁻⁴) | 34,3 ± 1,82 |
| Dans le même test, en utilisant le NGF, on obtient les résultats suivants: | |

| | % DE CELLULES A NEURITES |
|---|---|
| NGF: 1,6 nM | 51,7 ± 1,61 |
| Contrôle | 40,6 ± 2,08 |

Le mécanisme par lequel le Composé A exerce cette activité neurotrophique n'a pas été éclairci.

De toute façon on peut exclure qu'il implique un effet sérotoninergique parce que le Composé A ne montre aucune affinité pour les différents récepteurs de la sérotonine autres que le récepteur 5-HT_{1A} (c'est-à-dire les récepteurs 5-HT_{1B} , 5-HT_{1C} , 5-HT_{1D} , 5-HT₂ et 5-HT₃) et d'autre part des composés connus en tant que 5-HT_{1A} agonistes ou agonistes partiels, y compris la buspirone, l'ipsapirone et la 8-hydroxy-2-(di-n-propylamino)tétraline (8OH-DPAT), se sont revélés complètement inactifs dans le modèle décrit ci-dessus.

Le Composé A s'est aussi montré très actif (aux concentrations comprises entre 250 nM et 2,5 µM) dans un test de protection de la survie neuronale dans lequel les neurones sont placés dans un milieu très pauvre sans aucun facteur de croissance.

### Evaluation pharmacologique in vivo

Pour confirmer l'intérêt de ces résultats très positifs in vitro, on a mis au point un nouveau modèle expérimentai qui a permis l'évaluation in vivo de l'activité neurotrophique/neuroprotectrice des composés de formule (I) dans des processus de dégénérescence neuronale.

Un modèle expérimental pour ce type d'évaluation a été récemment proposé par Y. Nakagawa et al. (Brain Research, 1987, 408, 57-64).

Dans l'étude qu'ils ont conduite, ils ont mis en évidence la ressemblance entre les modifications neurochimiques et comportamentales, provoquées par injection d'un agent neurotoxique, notamment de colchicine, dans l'hippocampe et celles qu'on retrouve dans les patients arteints de la maladie d'Alzheimer. Sur la base des résultats publiés par Y. Nakagawa et en tenant compte des évidences bibliographiques du rôle de l'hippocampe dans les phénomènes mnémoniques on a essayé de mettre au point un modèle expérimental de meilleure faisabilité qui soit encore plus proche de la physiopathologie rencontrée dans la maladie d'Alzheimer.

Le modèle qu'on a mis en place répond aux questions suivantes: bonne faisabilité et lésions irréversibles, très spécifiques pour le système cholinergique septohippocampique.

En particulier, on a provoqué des lésions des neurones septaux par injection locale de vincristine en tant qu'inhibiteur de la polymérisation de la tubuline.

Par rapport aux autres produits de ce type (colchicine et vinblastine) et aux différents sites d'injection (intraventriculaire et intrahippocampique) qu'on a testés, on a ainsi obtenu les meilleurs résultats soit en terme de spécificité de blocage de la voie cholinergique septohippocampique soit en terme d'irréversibilité des lésions.

### PROTOCOLES EXPERIMENTAUX

### Méthode de production des lésions

Les animaux (rats mâles, Sprague Dawley d'environ 250 g) sont anesthésiés au pentobarbital (10 mg/kg i.p.), puis placés sur un appareil de stéréotaxie. En accord avec les coordonnées de l'atlas Paxinos et Watson, l'injection dans le spetum médian se fait selon le point de coordonnées suivantes:

| | |
|---|---|
| antériorité | 8,9 |
| latéralité | 0 |
| ventralité | 6,4 |
| (le point 0 correspond au lambda) | |

La vincristine est mise en solution dans un liquide céphalorachidien artificiel (ACSF) ayant la composition suivante;

| | |
|---|---|
| NaCl | 150 mM |
| CaCl₂ | 1,8 mM |
| MgSO₄ | 1,2 mM |
| K₂HPO₄ | 2 mM |
| glucose | 10 mM |
| pH 7,4 | |

à raison de 0,6 µmole de vincristine par ml.

1µl de cette solution (0,6 nmole de vincristine) est injecté localement dans le septum médian en 1 minute.

### Méthode d'évaluation des lésions

### - Evaluation morphologique (observation histoenzymologique de l'AchE)

Les animaux sont perfusés par l'aorte avec un mélange fixateur (glutaraldéhyde/paraformaldéhyde) à la vitesse de perfusion de 25 ml/min pendant 5 minutes.

Le cerveau est prélevé, la fixation poursuivie pendant 1 heure; cette étape est suivie par le lavage et la cryoprotection dans du saccharose à 20% dans un tampon phosphate. Le cerveau est alors coupé au cryostat, les coupes de 30 µm d'épaisseur sont prélevées au niveau du septum et de l'hippocampe et montées sur lame. Les lames sont incubées environ 15 heures dans le mélange suivant:
200 ml d'une solution stock: 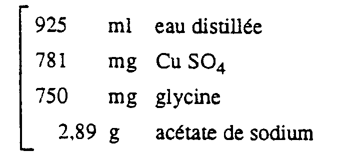
à laquelle on ajoute extemporanément
230 mg iodure d'acétylthiocholine
10 mg d'éthopropazine.
La réaction est ensuite révélée par passage dans du sulfure d'ammonium à 2% et amplifiée par AgNO₃ à 0,25 %.

Les sites à acétylcholinestérases (en général associés aux synapses cholinergiques) sont révélés sous forme d'un précipité brun.

### - Evaluation biochimique (détermination de l'activité ChAT)

La détermination de l'activité ChAT a été conduite selon la méthode de Fonnum (J. Neurochem., 24, 1975, 407-409). Les tissus sont homogénéises dans un potter (à 4°C). Chaque échantillon est ramené à 1 mg de protéines/ml. 10 µl d'homogénat sont incubés en présence de 1,5 mM de choline, 70 µM d'acétylCoA, 30 µM d'acétylCoA marqué au ¹⁴C, en présence d'ésérine 0,15 mM. L'incubation dure 7 minutes 30 sec. à 37°C. L'arrêt de la réaction se fait au bain de glace avec 5 ml de tampon phosphate. Après addition de 2 ml de tétraphénylbore/acétone et de 5 ml de scintillant, l'acétylcholine marquée au ¹⁴C est comptée au compteur à scintillations. Chaque échantillon est dosé trois fois. Chaque lot est comparé au lot contrôle correspondant par le test de Student.

### - Evaluation comportamentale

Cette étude porte sur des groupes d'animaux réservés à cet effet, les rats étant mis en cycle inversé. Les rats utilisés dans ce cas sont de souche Wistar, lésés selon la même méthode décrite ci-dessus.

### - Mémoire sociale

(A.Perio et al.. Psychopharmacology, 1989, 97, 262-268). Selon ce test, un rat juvénile est mis dans la cage d'un rat adulte et la durée de la relation sociale est mesurée en secondes (T1). Les animaux sont séparés pendant 15 minutes et le même rat juvénile lui est présenté à nouveau. On mesure de la même façon le temps de contact T2. Dans le cas d'animaux normaux, le souvenir de la première rencontre se traduit par un rapport T2/T1<1. Dans le cas d'une altération de mémoire le rapport est T2/T1≧1.

### - Test du labyrinthe en T

Conduit selon la méthode décrite par P. Soubrié et al., J. Pharmacol. (Paris), 1977, 8, 3, 393-403 pour le test du Labyrinthe en Y.

### - Test de la planche à trous

(S.E. File et al., Pharmacol. Biochem. Behav., 1985, 22, 941-944).

### Evaluation des lésions

L'administration intraseptale de vincristine entraîne une chute rapide (une semaine après l'injection) et importante (de 60 à 70%) des marqueurs cholinergiques dans l'hippocampe [choline acétyltransférase (ChAT) et acétylcholinestérase (AchE)] ainsi qu'une dégénérescence des neurones du septum médian, qui se révèle maximale deux semaines après l'injection, et qui est associée à la diminution des marqueurs cholinergiques. Cette dégénérescence semble être irréversible, puisqu'on la retrouve trois mois après l'injection de vincristine, et entraîne des altérations fonctionnelles.

Parmi les altérations fonctionnelles consécutives à l'administration de vincristine, on a relevé principalement un déficit très important et inréversible des fonctions mnémoniques (test de mémoire sociale). Des évaluations parallèles ont mis en évidence, entre autres, une réduction des capacités d'exploration ("Labyrinthe en T" et "Planche à trous").

### TRAITEMENTS

Dans ce modèle, on a évalué les effets de l'administration du Composé A aux animaux lésés en comparaison aux effets obtenus avec le NGF.

Le Composé A est administré, per os, 2 à 3 heures après l'injection de vincristine, en suspension dans la carboxyméthylcellulose à 1%, à raison de 10 ml de suspension par kg. Le lot contrôle est traité avec le véhicule. Le traitement est chronique, le produit est administré une fois par jour pendant 11 jours. Le Composé A a été administré à trois doses différentes: 2,5 mg/kg, 5 mg/kg et 10 mg/kg à des groupes de 8 animaux chacun, et 24 heures après l'arrêt du traitement les animaux sont sacrifiés.

Le NGF, au contraire, est administré, par perfusion intraventriculaire en solution dans le ACSF, contenant de l'albumine de rat à raison de 0,01 % et de la gentamycine (1 ml/15 ml), selon la méthode décrite par W. Fisher et al., Nature, 1987, 329 (6134), 65-68. La concentration en NGF de la solution est calculée de telle sorte qu'en fonction du débit de diffusion (0,44 ± 0,02 µl/h) les animaux (7 rats) reçoivent 0,105 µg, 1,05 µg, ou 10,5 µg de NGF en dose totale sur deux semaines de perfusion. Pour le lot contrôle, le NGF est remplacé par une protéine de poids moléculaire équivalent (environ 130.000) ne possédant aucune activité neurotrophique: le cytochrome C. Deux semaines après la lésion et le début du traitement, les animaux sont sacrifiés. Un groupe d'animaux sacrifiés est perfusé pour une évaluation histoenzymologique de l'AChE, les autres animaux sont utilisés pour effectuer un dosage ChAT dans l'hippocampe et le septum.

### RESULTATS

### Evaluation morphologique

Chez les animaux lésés et non traités, on n'observe presque aucun précipité brun tandis que, chez les animaux traités avec 5 mg/kg du Composé A, les bourgeonnements hippocampiques observés donnent une image de la structure proche de celle observée chez un animal normal. On obtient ainsi des résultats similaires à ceux obtenus avec le NGF.

### Evaluation biochimique

La lésion entraîne une chute très significative de l'activité ChAT dans l'hippocampe qui augmente en fonction de la dose du Composé A administré jusqu'à une récupération totale à la dose de 10 mg/kg, comparable à la récupération qu'on obtient dans le groupe d'animaux perfusés avec le NGF. Les résultats sont reportés dans le tableau suivant

**TABLEAU II**

| | Activité ChAT (pmole/mg/mn) |
|---|---|
| Animaux normaux non lésés | 277 ± 13 |
| Contrôles lésés | 115 ± 18 |
| NGF 0,105 µg/rat/2 semaines | 168 ± 27 |
| NGF 1,05 µg/rat/2 semaines | 336 ± 28 |
| NGF 10,5 µg/rat/2 semaines | 293 ± 10 |
| Animaux normaux non lésés | 242 ± 19 |
| Contrôles lésés | 97 ± 18 |
| Composé A 2,5 mg/kg/jour | 164 ± 42 |
| Composé A 5 mg/kg/jour | 204 ± 24 |
| Composé A 10 mg/kg/jour | 242 ± 27 |

Dans le septum, la lésion entraîne une chute de l'activité ChAT dont la récupération est en fonction de la dose du Composé A administré. Dans le cas d'animaux perfusés avec le NGF les résultats ne sont pas significatifs; ceci peut être dû au fait qu'à la nécrose septale associée à l'injection de vincristine se surajoute une nécrose supplémentaire consécutive à la canulation dans un ventricule proche du septum.

### Evaluation comportamentale

### - Mémoire sociale

Une fois lésés avec la vincristine, les animaux présentent des altérations de la mémoire sociale qui semblent irréversibles (elles sont encore présentes 50 jours après l'injection de vincristine). Le Composé A a été essayé à la dose de 10 mg/kg per os en comparaison avec le NGF à la dose de 10,5 µg et on a effectué ce test au 7ème jour de la lésion. Dans les deux cas, on a observé un effet protecteur très important en obtenant un rapport T2/T1 compris entre 0,6 et 0,7.

### - Labyrinthe en T

Les valeurs obtenues chez les animaux lésés et non traités sont significatives d'une altération des capacités d'exploration qui par contre sont récupérées à un niveau normal par traitement avec le Composé A à la dose de 10 mg/kg. Ce test a été effectué en aveugle, 7 jours après l'arrêt du traitement.

### - Planche à trous

Dans le lot contrôle, la majorité (6 rats) des animaux ont perdu toute capacité d'exploration, seuls deux rats montrent une hyperactivité exploratoire. Le traitement avec le Composé A à la dose de 10 mg/kg apporte une normalisation du comportement exploratoire en se référant à des valeurs moyennes d'animaux normaux. Ce test a été effectué, également en aveugle, 11 jours après l'arrêt des traitements.

Compte tenu des résultats obtenus avec ce modèle, les composés de formule (I) ainsi que leurs sels d'addition pharmaceutiquement acceptables sont utilisables dans le traitement et/ou la prophylaxie de toutes les maladies qui impliquent une dégénérescence neuronale. Plus particulièrement, les composés de formule (I) ainsi que leurs sets d'addition pharmaceutiquement acceptables sont utilisables notamment dans les indications suivantes: troubles de la mémoire, démence vasculaire, troubles post-encéphalitiques, troubles post-apoplectiques, syndromes post-traumatiques dus à un traumatisme crânien, troubles dérivant d'anoxies cérébrales, maladie d'Alzheimer, démence sénile, démence subcorticale, telle que la chorée de Huntington et la maladie de Parkinson, démence provoquée par le SIDA, neuropathies dérives de morbidité ou dommage des nerfs sympathiques ou sensoriels, et maladies cérébrales, telles que l'oedème cérébral, et les dégénérescences spinocérébelleuses.

L'administration, des composés de formule (I) ou de leurs sels d'addition pharmaceutiquement acceptables peut être convenablement effectuée par voie orale, parentérale, sublinguale ou transdermique. La quantité de principe actif à administrer dans le traitement des troubles cérébraux et neuronaux selon la méthode de la présente invention dépend, comme toujours, de la nature et de la gravité des affections à traiter ainsi que du poids des malades. Néanmoins, les doses unitaires préférées comprendront généralement de 2 à 300 mg, allant de préférence de 5 à 150 mg, comprises par exemple entre 5 et 50 mg, à savoir 5, 10, 20, 30, 40 et 50 mg, de produit. Ces doses unitaires seront administrées normalement une ou plusieurs fois par jour, par exemple 2, 3, 4, ou 5 fois par jour, de préférence une à trois fois par jour, la dose globale chez l'homme étant variable entre 2 et 900 mg par jour, par exemple de 3 à 500 mg, plus convenablement de 10 à 300 mg par jour.

Pour un emploi thérapeutique et/ou prophylactique selon la présente invention, les composés de formule (I) et leurs sels d'addition pharmaceutiquement acceptables sont de préférence élaborés dans des compositions pharmaceutiques. Les compositions pharmaceutiques de l'invention renferment une quantité efficace pour le traitement ou la prophylaxie des troubles cérébraux et neuronaux d'au moins un produit choisi parmi les composés de formule (I) et leurs sels d'addition pharmaceutiquement acceptables, en association avec un véhicule pharmaceutique inerte.

En ce qui concerne l'administration par voie orale ou sublinguale, on utilise en particulier des comprimés, simples ou dragéifiés, des gélules granulées éventuellement à libération retardée, des gouttes ou encore des liposomes. En ce qui concerne l'administration par voie intraveineuse, sous-cutanée ou intramusculaire, on recourt à des solutions stériles ou stérilisables, tandis que l'on peut utiliser des patches conventionnels pour l'administration par voie transdermique.

Les compositions pharmaceutiques selon la présente invention peuvent être préparées selon des méthodes usuelles, telles que celles décrites dans EP-101381 ou dans le Remington's Pharmaceutical Sciences, 18th Ed., Mack Publ. Co. Le principe actif peut y être incorporé à des excipients habituellement employés dans ces compositions pharmaceutiques, tels que le talc, la gomme arabique, le lactose, l'amidon, le stéarate de magnésium, les véhicules aqueux ou non, les corps gras d'origine animale ou végétale, les dérivés paraffiniques, les glycols, les différents agents mouillants, dispersants, ou émulsifiants, les conservateurs, etc.

Les compositions pharmaceutiques de l'invention peuvent avantageusement contenir un composé de formule (I) ou un de ses sels d'addition pharmaceutiquement acceptables en association avec un ou plusieurs autres médicaments connus et communément utilisés dans les mêmes indications thérapeutiques.

## Revendications

1. Utilisation d'au moins un composé de formule (I) 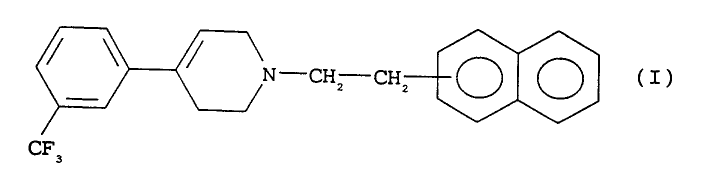 ou d'un de ses sels d'addition avec des acides pharmaceutiquement acceptables pour la préparation de médicaments destinés au traitement et/ou à la prophylaxie des maladies cérébrales et neuronales.

2. Utilisation selon la revendication 1 pour la préparation de médicaments destinés au traitement et/ou à la prophylaxie des maladies qui impliquent une dégénérescence neuronale.

3. Utilisation selon la revendication 2 pour la préparation de médicaments destinés au traitement et/ou à la prophylaxie des troubles de la mémoire, de la démence vasculaire, des troubles post-encéphalitiques, des troubles post-apoplectiques, des syndromes post-traumatiques dûs à un traumatisme crânien, des troubles dérivant d'anoxies cérébrales, de la maladie d'Alzheimer, de la démence sénile, de la démence subcorticale telle que la chorée de Huntington et la maladie de Parkinson, de la démence provoquée par le SIDA, des neuropathies dérivées de la morbidité ou du dommage des nerfs sympatiques ou sensoriels, de l'oedème cérébral ou des dégénérescences spinocérébelleuses.

4. Utilisation selon la revendication 3 pour la préparation de médicaments destinés au traitement de la démence vasculaire, de la démence sénile ou de la maladie d'Alzheimer.

5. Utilisation selon une quelconque des revendications 1 à 4, dans laquelle le composé de formule (I) est choisi entre la 1-[2-(2-naphtyl)éthyl]-4-(3-trifluorométhylphényl)-1,2,3,6-tétrahydropyridine et ses sels d'addition avec des acides pharmaceutiquements acceptables.

6. Utilisation selon une quelconque des revendications 1 à 5, où le médicament est une composition pharmaceutique sous forme de dosage unitaire.

7. Utilisation selon la revendication 6, dans laquelle la forme de dosage unitaire comprend de 2 à 300 mg du principe actif.

8. Utilisation selon la revendication 7, dans laquelle la forme de dosage unitaire comprend de 5 à 150 mg du principe actif.

9. Utilisation selon la revendication 8, dans laquelle la forme de dosage unitaire comprend de 5 à 50 mg du principe actif.

## Claims

1. Use of at least one compound of formula (I) 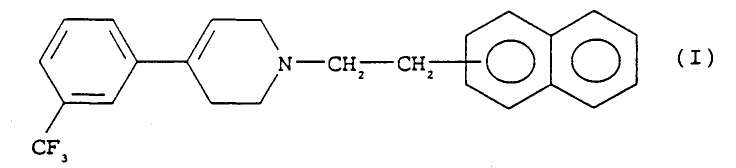 or of a pharmaceutically acceptable acid addition salt thereof for the preparation of medicaments intended for the treatment and/or the prophylaxis of cerebral and neuronal diseases.

2. Use according to claim 1 for the preparation of medicaments intended for the treatment and/or the prophylaxis of diseases which involve neuronal degeneration.

3. Use according to claim 2 for the preparation of medicaments intended for the treatment and/or the prophylaxis of memory disorders, vascular dementia, post-encephalitic disorders, post-apoplectic disorders, post-traumatic syndromes caused by injury to the heed, disorders associated with cerebral anoxia, Alzheimer's disease, senile dementia, sub-cortical dementia, such as Huntington's chorea and Parkinson's disease, AIDS dementia, neuropathies caused by morbidity or lesions of the sympathetic or sensory nerves, cerebral oedema or spinocerebellar degenerations.

4. Use according to claim 3 for the preparation of medicaments intended for the treatment of vascular dementia, senile dementia or Alzheimer's disease.

5. Use according to any one of claims 1 to 4, in which the compound of formula (I) is chosen from 1-[2-(2-naphthyl)ethyl]-4-(3-trifluoromethylphenyl)-1,2,3,6-tetrahydropyridine and the pharmaceutically acceptable acid addition salts thereof.

6. Use according to any one of claims 1 to 5, where the medicament is a pharmaceutical composition in unit dosage form.

7. Use according to claim 6, in which the unit dosage form comprises from 2 to 300 mg of active principle.

8. Use according to claim 7, in which the unit dosage form comprises from 5 to 150 mg of active principle.

9. Use according to claim 8, in which the unit dosage form comprises from 5 to 50 mg of active principle.

## Patentansprüche

1. Verwendung zumindest einer Verbindung der Formel (I) 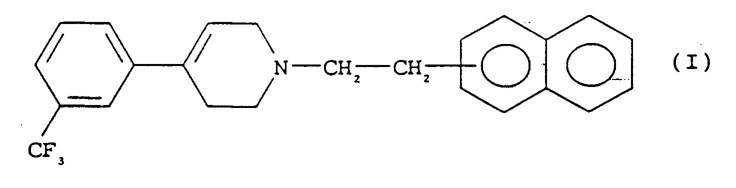 oder eines ihrer Additionssalze mit pharmazeutisch annehmbaren Säuren bei der Herstellung von Medikamenten zur Behandlung und/oder Prophylaxe zerebraler und neuronaler Erkrankungen.

2. Verwendung nach Anspruch 1 bei der Herstellung von Medikamenten zur Behandlung und/oder Prophylaxe von Erkrankungen, die eine neuronale Degeneration implizieren.

3. Verwendung nach Anspruch 2 bei der Herstellung von Medikamenten zur Behandlung und/oder Prophylaxe von Gedächtnisstörungen, vaskulärer Demenz, postenzephalitischen Störungen, postapoplektischen Störungen, posttraumatischen Syndromen aufgrund von Schädeltraumata, Störungen, die auf zerebrale Anoxien zurückzuführen sind, Alzheimer-Krankheit, seniler Demenz, subkortikaler Demenz, wie Chorea Huntington und Parkinson-Syndrom, durch AIDS hervorgerufener Demenz, Neuropathien, die auf Morbidität oder Schädigungen sympathischer oder sensorischer Nerven zurückzuführen sind, Gehirnödem oder das Rückenmark und Kleinhirn betreffenden Degenerationen.

4. Verwendung nach Anspruch 3 bei der Herstellung von Medikamenten zur Behandlung von vaskulärer Demenz, seniler Demenz oder Alzheimer-Krankheit.

5. Verwendung nach einem der Ansprüche 1 bis 4, wobei die Verbindung der Formel (I) ausgewählt wird aus 1-[2-(2-Naphthyl)ethyl]-4-(3-trifluormethylphenyl)-1,2,3,6-tetrahydropyridin und seinen Additionssalzen mit pharmazeutisch annehmbaren Säuren.

6. Verwendung nach einem der Ansprüche 1 bis 5, wobei das Medikament eine pharmazeutische Zusammensetzung in Einheitsdosierungsform ist.

7. Verwendung nach Anspruch 6, wobei die Einheitsdosierungsform 2 bis 300 mg des aktiven Bestandteils umfaßt.

8. Verwendung nach Anspruch 7, wobei die Einheitsdosierungsform 5 bis 150 mg des aktiven Bestandteils umfaßt.

9. Verwendung nach Anspruch 8, wobei die Einheitsdosierungsform 5 bis 50 mg des aktiven Bestandteils umfaßt.
